# EUROPEAN PATENT APPLICATION

(11) **EP 3 339 292 A1**
(43) Date of publication of application: **27.06.2018**
(21) Application number: 16460095.9
(22) Date of filing: 23.12.2016
(51) Int. Cl.: C07D 209/48, A61K 31/4035, A61P 29/00

(54) **COCRYSTALS OF APREMILAST**

(71) Applicant: Zaklady Farmaceutyczne Polpharma SA, 83-200 Starogard Gdanski (PL)
(72) Inventor: ORACZ, Monika, 97-360 Kamiensk (PL); SKOCZEN, Przemyslaw, 83-010 Straszyn (PL)
(74) Representative: Obrycki, Pawel Andrzej

(57) **Abstract**

The invention relates to cocrystals of N-[2-[(1S)-1-(3-ethoxy-4-methoxyphenyl)-2-(methylsulfonyl)ethyl]-1,3-dioxo-2,3-dihydro-1H-isoindol-4-yl]acetamide (Apremilast) with caffeine (APM-CAF) and 4-hydroxybenzoic acid (APM-4HB). It also refers to pharmaceutical compositions and dosage forms comprising said cocrystals. The invention also concerns said pharmaceutical compositions or dosage forms for use in a method of treating a disease or disorder defined in the claims. Finally, the invention pertains to the use of said cocrystals for the preparation of pharmaceutical compositions and dosage forms.

## Description

The invention relates to cocrystals of Apremilast and pharmaceutical compositions/dosage forms containing same. It also refers to the use of said cocrystals for the preparation of a pharmaceutical composition or dosage form. The invention also concerns said pharmaceutical compositions or dosage forms for use in a method of treating a disease or disorder as defined in the claims.

### Background art

Apremilast, i.e. N-[2-[(1S)-1-(3-ethoxy-4-methoxyphenyl)-2- (methylsulfonyl)ethyl]-1,3-dioxo-2,3-dihydro-1H-isoindol-4-yl]acetamide, also referred to herein as "APM", has the following chemical formula (Formula 1), wherein "S", if not connected via a chemical bond in the formula, denotes the S-form of the chiral center.

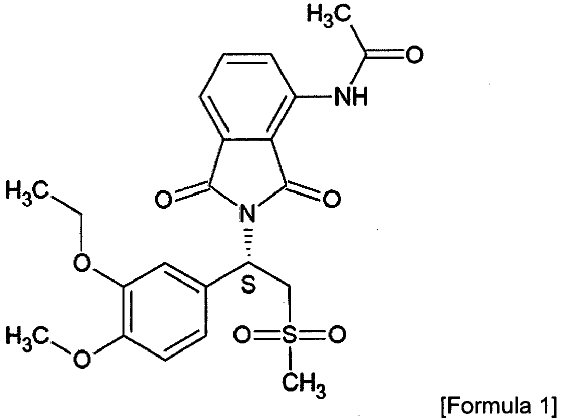

Apremilast is a phosphodiesterase 4 inhibitor and blocks the action of an enzyme inside cells called phosphodiesterase 4 (PDE4). This enzyme plays a role in triggering the production of messenger molecules in the immune system called cytokines, which are involved in inflammation and other processes that cause psoriasis and psoriatic arthritis. By blocking PDE4, Apremilast reduces the level of these cytokines in the body, and so reduces the inflammation and other symptoms of psoriasis and psoriatic arthritis. Apremilast is the active ingredient of the medicament Otezla®, distributed by Celgene, Ltd, for the treatment of adults with psoriatic arthritis (inflammation of the joints associated with psoriasis) and moderate to severe plaque psoriasis (a disease causing red, scaly patches on the skin).

EP2276483 B1 discloses various polymorphic forms of Apremilast, i.e. Forms A, B, C, D, E, F and G. Regarding the characterization of these Forms A, B, C, D, E, F and G, in particular their XRPD peak locations and intensity, reference is made to EP2276483 B1. The stability, interconversion and equilibration studies in EP2276483 B1 show that most of these crystalline forms have a high tendency of interconversion, i.e. one polymorphic form converts into another polymorphic form in the presence of solvents. Although Form B is the commercially available form of Apremilast (Otezla®) and has quite good properties, there is a constant need for improvement of existing dosage forms.

Accordingly, one objective underlying the present invention was to improve the existing dosage forms and existing forms of Apremilast.

### Summary of the invention

The invention relates to cocrystals of N-[2-[(1S)-1-(3-ethoxy-4-methoxyphenyl)-2-(methylsulfonyl)ethyl]-1,3-dioxo-2,3-dihydro-1H-isoindol-4-yl]acetamide with caffeine (APM-CAF) and 4-hydroxybenzoic acid (APM-4HB). It also refers to pharmaceutical compositions and dosage forms comprising said cocrystals. The invention also concerns said pharmaceutical compositions or dosage forms for use in a method of treating a disease or disorder defined in the claims. Finally, the invention pertains to the use of said cocrystals for the preparation of pharmaceutical compositions and dosage forms.

### List of figures

- Figure 1:: shows the preparation of N-[2-[(1S)-1-(3-ethoxy-4-methoxyphenyl)-2-(methylsulfonyl)ethyl]-1,3-dioxo-2,3-dihydro-1H-isoindol-4-yl]acetamide (APM).

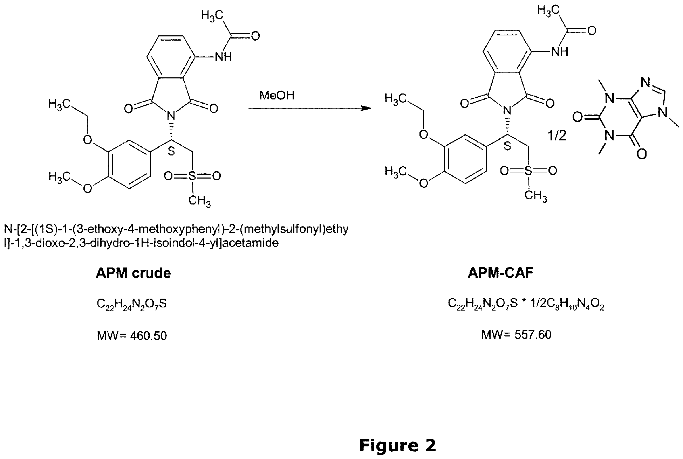

- Figure 2:: shows the cocrystallization of N-[2-[(1S)-1-(3-ethoxy-4-methoxyphenyl)-2-(methylsulfonyl)ethyl]-1,3-dioxo-2,3-dihydro-1H-isoindol-4-yl]acetamide (APM) with caffeine.
- Figure 3:: shows a representative XRPD pattern of Apremilast and caffeine cocrystal.
- Figure 4:: shows a representative IR spectrum of Apremilast and caffeine cocrystal.
- Figure 5:: shows a representative DSC plot of Apremilast and caffeine cocrystal.
- Figure 6:: shows a representative TGA plot of Apremilast and caffeine cocrystal.
- Figure 7:: shows a representative DVS plot of Apremilast and caffeine cocrystal.
- Figure 8:: shows the dissolution profile of a cocrystal of Apremilast with caffeine compared with that of prior art Form B of Apremilast.

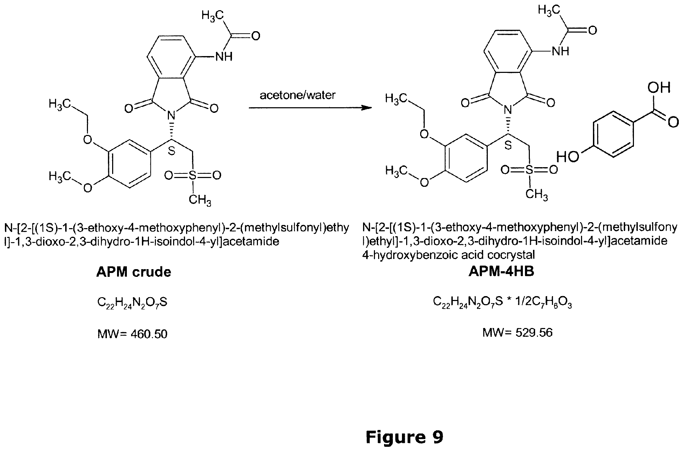

- Figure 9:: shows the cocrystallization of N-[2-[(1S)-1-(3-ethoxy-4-methoxyphenyl)-2-(methylsulfonyl)ethyl]-1,3-dioxo-2,3-dihydro-1H-isoindol-4-yl]acetamide (APM) with 4-hydroxybenzoic acid.
- Figure 10:: shows a representative XRPD pattern of Apremilast and 4-hydroxybenzoic acid cocrystal.
- Figure 11:: shows a representative IR spectrum of Apremilast and 4-hydroxybenzoic acid cocrystal.
- Figure 12:: shows a representative DSC plot of Apremilast and 4-hydroxybenzoic acid cocrystal.
- Figure 13:: shows a representative TGA plot of Apremilast and 4-hydroxybenzoic acid cocrystal.
- Figure 14:: shows a representative DVS plot of Apremilast and 4-hydroxybenzoic acid cocrystal.
- Figure 15:: shows the dissolution profile of cocrystal of Apremilast with 4-hydroxybenzoic acid compared with that of prior art Form B of Apremilast.
- Figure 16:: shows the results of ¹³C CP/MAS NMR experiments with Apremilast, 4-hydroxybenzoic acid and cocrystals of APM and 4-hydroxybenzoic acid (Figure 16a: pure Apremilast Figure 16b: 4-hydroxybenzoic acid, Figure 16c: cocrystals of Apremilast and 4-hydroxybenzoic acid).
- Figure 17:: shows the results of ¹³C CP/MAS NMR experiments with Apremilast, caffeine and cocrystals of APM and caffeine (Figure 17a: pure apremilast Figure 17b: caffeine, Figure 17c: cocrystals of Apremilast and caffeine).

### Detailed description

It has unexpectedly been found that it is possible to provide cocrystals of Apremilast with caffeine and 4-hydroxybenzoic acid. In general, the successful formation of cocrystals is neither predictable nor otherwise guaranteed (see Experiment 15, summarizing some cocrystallization experiments). Furthermore, computational analysis cannot predict the structure or properties of cosolvates. Of particular importance is the fact that cocrystal formation is a new field where there is generally not yet much experience and knowledge available in the art. As such, general trends are not yet evident. It is also expected that the change in properties when comparing a drug polymorph with cocrystals will be far more drastic as compared with the variance in properties amongst polymorphs. This means that the chances of success for providing cocrystals having properties comparable to existing polymorphs, or even improved cocrystals, in the present case were generally low.

It can also be expected that processes for producing cocrystals, in particular in large scale applications, will require different approaches as have been developed in the field of polymorphs. At present the number of drug cocrystals in pharmaceutics on the market, if any, is low.

The cocrystals described herein may have various benefits over the known forms of Apremilast, in particular other known crystalline forms.

The cocrystals of Apremilast with caffeine (1,3,7-Trimethylxanthine or 1,3,7-Trimethylpurine-2,6-dione) are stable, substantially non-solvated and slightly hygroscopic. They also have good flow characteristics. In particular, the cocrystals of Apremilast with caffeine have a superior dissolution profile, in particular a higher dissolution rate, in buffer pH 6.8 + 0.3% SLS, particularly compared to prior art Form B of Apremilast.

The cocrystals of Apremilast with 4-hydroxybenzoic acid are stable, substantially non-solvated and have good flow characteristics. In particular, the cocrystals of Apremilast with 4-hydroxybenzoic acid have a superior dissolution profile, in buffer pH 6.8 + 0.3% SLS, particularly compared to prior art Form B of Apremilast.

Since Form B is the commercially available form of Apremilast (Otezla®), benefits shown by the present cocrystals versus Form B demonstrate the unexpected improvements provided by the present application. In particular, a comparison with Form B is of great interest, since the marketed drug form generally represents the "gold standard" for any further pharmaceutical development.

Accordingly, the objective underlying the present invention has been solved.

The present invention thus refers to cocrystals of N-[2-[(1S)-1-(3-ethoxy-4-methoxyphenyl)-2-(methylsulfonyl) ethyl]-1,3-dioxo-2,3-dihydro-1H-isoindol-4-yl]acetamide (Apremilast). In particular, it relates to cocrystals of Apremilast and caffeine (1,3,7-trimethylpurine-2,6-dione) or 4-hydroxybenzoic acid.

Within the meaning of the present invention, the term "solvent" refers to any solvent such as water and organic or inorganic solvents.

The term "room temperature" as used herein is understood to mean temperatures of about 15 °C to about 25 °C.

Herein, the X-ray powder diffraction peaks of the cocrystals are described by their 2theta angles with a tolerance of "±0.2". In the context of the present invention, it is also possible to use a smaller tolerance of "±0.1" or even "±0.0".

The term "cocrystal" denotes one or more crystals of the same type, i.e. one or more crystals which are characterized by the same components, e.g., APM and caffeine, and the same crystal structure. In other words, the term "cocrystal" is not meant to be limited to one single piece of crystal. The term "cocrystal" refers to a crystalline form of a substance which comprises the drug component and at least one further component which is not a solvent, e.g., APM and caffeine. That is, the term "cocrystal" does not refer to cosolvates. The term "cocrystals" does not refer to a mixture of crystals of APM and crystals of the further compound (e.g., caffeine).

Regarding the synthesis of Apremilast, reference is made to Example 1 herein, as well as to EP2276483 B1 and US 6,962,940. Apremilast as the starting material for crystallizing the cocrystals of the present invention can be prepared by using any method.

The invention is further described with respect to the following items:

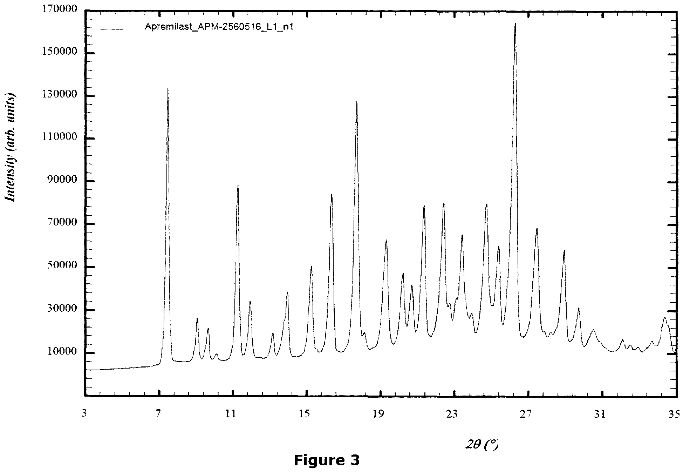

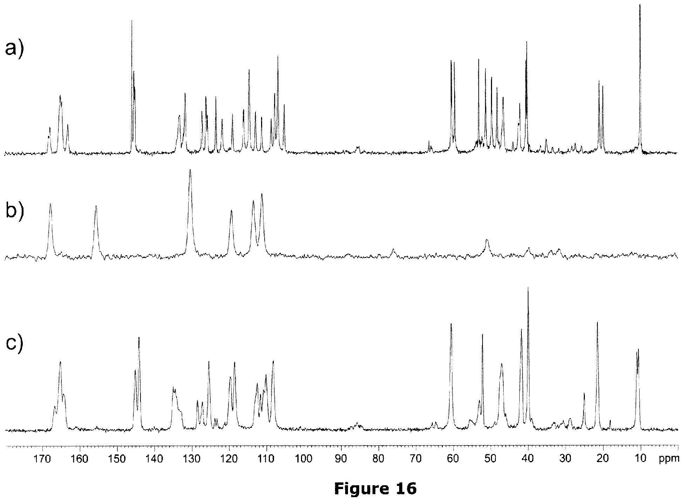

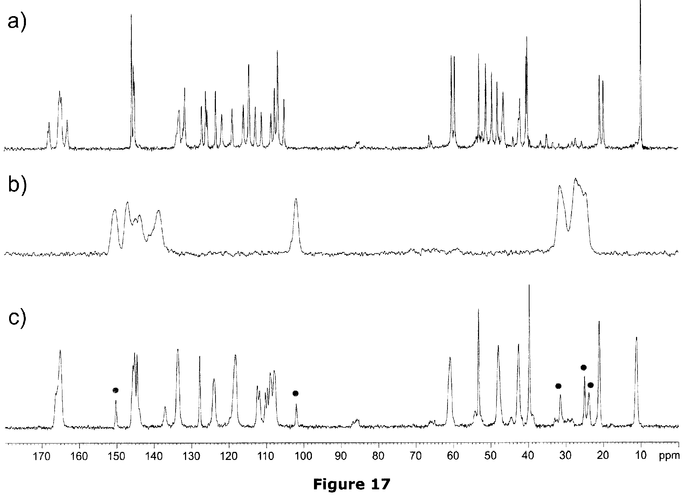

In one aspect, the invention refers to Apremilast-caffeine cocrystals (APM-CAF):
C1. Cocrystal of N-[2-[(1 S)-1-(3-ethoxy-4-methoxyphenyl)-2-(methylsulfonyl) ethyl]-1,3-dioxo-2,3-dihydro-1H-isoindol-4-yl]acetamide and 1,3,7-trimethylpurine-2,6-dione (caffeine).
C2. The cocrystal of item C1, wherein the molar ratio of N-[2-[(1S)-1-(3-ethoxy-4-methoxyphenyl)-2-(methylsulfonyl) ethyl]-1,3-dioxo-2,3-dihydro-1H-isoindol-4-yl]acetamide to 1,3,7-trimethylpurine-2,6-dione is about 1:2. That is, the ratio of of N-[2-[(1S)-1-(3-ethoxy-4-methoxyphenyl)-2-(methylsulfonyl) ethyl]-1,3-dioxo-2,3-dihydro-1H-isoindol-4-yl]acetamide to 1,3,7-trimethylpurine-2,6-dione can, e.g., be from 0.8:2 to 1.2:2. The ratio can be determined by using gaschromatographic analysis, quantitative 13C NMR measurement, or high performance liquid chromatography (HPLC) (see Figures 16 and 17).
C3. The cocrystal of item C1 or C2, having an X-ray powder diffraction pattern with peaks at 2-theta angles of 7.5±0.2 degrees 2theta, 11.3±0.2 degrees 2theta, 16.3±0.2 degrees 2theta, 17.7±0.2 degrees 2theta, 19.3±0.2 degrees 2theta, 21.4±0.2 degrees 2theta, 22.4±0.2 degrees 2theta, 24.7±0.2 degrees 2theta, 26.3 ±0.2 degrees 2theta, and 27.5 ±0.2 degrees 2theta when using Cu-Kα radiation. Details regarding the X-ray powder diffraction measurement are provided herein below.
C4. The cocrystal of any preceding items,
(i) having a single endothermic event in a differential scanning calorimeter plot, preferably with a peak value at 148.52±6 °C, further preferred at 148.52±4 °C, even more preferred at 148.52±2 °C; and/or
(ii) wherein the X-ray powder diffraction pattern has further peaks at 2-theta angles of 9.1±0.2 degrees 2theta, 11.9±0.2 degrees 2theta, 14.0±0.2 degrees 2theta, 15.3±0.2 degrees 2theta, 20.2±0.2 degrees 2theta, 20.7±0.2 degrees 2theta, 23.4±0.2 degrees 2theta, 25.4±0.2 degrees 2theta, and 28.9±0.2 degrees 2theta, when using Cu-Kα radiation; and/or
(iii) which is substantially polymorphically pure, and preferably contains less than 5 wt.-% of other crystalline, non-crystalline or amorphous forms of N-[2-[(1S)-1-(3-ethoxy-4-methoxyphenyl)-2- (methylsulfonyl)ethyl]-1,3-dioxo-2,3-dihydro-1H-isoindol-4-yl]acetamide, 1,3,7-trimethylpurine-2,6-dione, and cocrystals thereof.
The applied temperature range for the differential scanning calorimeter measurement is 25-250°C. Polymorphic purity can, e.g., be determined by X-ray powder diffraction analysis as is known in the art. Details regarding the differential scanning calorimeter measurement are provided herein below.
C5. The cocrystal of any preceding items, having an X-ray powder diffraction pattern, wherein the peaks recited in item C3 represent the peaks with highest intensity, and/or the peak at 26.3±0.2 degrees 2theta has the highest intensity (set to 100).
C6. The crystalline form of item C4(ii), wherein the X-ray powder diffraction pattern is free of further peaks having an intensity which is higher than the intensity of the peak at 9.1±0.2 degrees 2theta.
C7. The cocrystal of any preceding item, wherein said X-ray powder diffraction pattern is free of further peaks which have an intensity which is in the range of intensities of the peaks defined in items C3 and C4, in particular the X-ray powder diffraction pattern is free of further peaks having an intensity which is higher than the intensity of the peak at 11.9±0.2 degrees 2theta.
C8. The cocrystal of any preceding item, which is substantially pure, i.e. contains less than 1 area-%, or less than 0.5 area-%, preferably less than 0.1 area-%, of compounds other than N-[2-[(1S)-1-(3-ethoxy-4-methoxyphenyl)-2- (methylsulfonyl)ethyl]-1,3-dioxo-2,3-dihydro-1H-isoindol-4-yl]acetamide and 1,3,7-trimethylpurine-2,6-dione as determined by quantitative ¹³C NMR measurement or high performance liquid chromatography (HPLC).
C9. The cocrystal of any preceding item, having a mass loss of less than 0.5% of the total mass of the sample in a thermogravimetric analysis (TGA) upon heating from 25°C to 160°C with the gradient of 7°C/min. Details regarding the TGA measurement are provided herein below.
C10. The cocrystal of any preceding item, having an X-ray powder diffraction pattern with peaks and/or intensities as shown in Table 1 (below) or Figure 3.
C11. The cocrystal of any preceding item, having peaks at 2-theta angles ±0.2 degrees 2theta as shown in the following: 7.5, 9.1, 9.7, 11.3, 11.9, 13.2, 14.0, 15.3, 16.3, 17.7, 19.3, 20.2, 20.7, 21.4, 22.4, 23.4, 24.7, 25.4, 26.3, 27.5, 28.9, 29.7, 30.4, and 33.6.
C12. The cocrystal of any preceding item, wherein the peaks recited in item C3 and/or item C4 have intensities or preferred intensities as shown in the following Table 1, preferably wherein the X-ray powder diffraction pattern shows all peaks (±0.2 degrees 2theta) and all intensities or preferred intensities as shown in the following Table 1:

**Table 1.**

| **2-theta(deg)** | **Rel. int. I(a.u.)** | **Preferred intensities Rel. int. I(a.u.)** |
|---|---|---|
| 7.5 | 50-80 | about 65 |
| 9.1 | 7-14 | about 10 |
| 9.7 | 3-13 | about 8 |
| 11.3 | 30-60 | about 45 |
| 11.9 | 10-20 | about 15 |
| 13.2 | 2-9 | about 6 |
| 14.0 | 15-35 | about 22 |
| 15.3 | 18-38 | about 27 |
| 16.3 | 35-65 | about 49 |
| 17.7 | 60-90 | about 75 |
| 19.3 | 23-44 | about 34 |
| 20.2 | 10-21 | about 16 |
| 20.7 | 7-18 | about 12 |
| 21.4 | 20-43 | about 33 |
| 22.4 | 22-46 | about 34 |
| 23.4 | 15-30 | about 21 |
| 24.7 | 23-45 | about 35 |
| 25.4 | 13-33 | about 20 |
| 26.3 | 100 | 100.00 |
| 27.5 | 28-48 | about 37 |
| 28.9 | 15-44 | about 31 |
| 29.7 | 5-14 | about 9 |
| 30.4 | 2-9 | about 6 |
| 33.6 | 2-9 | about 6 |

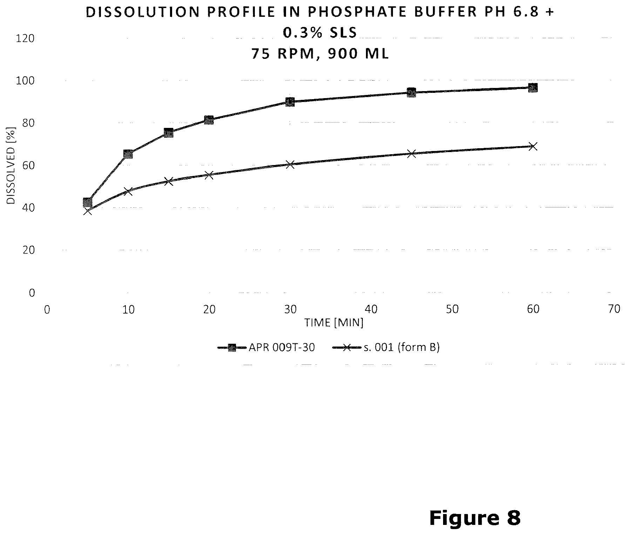

C13. The cocrystal of any preceding item, having an infrared spectrum comprising peaks at wavenumbers of 3369 ± 2 cm⁻¹, 2981 ± 2 cm⁻¹, 2939 ± 2 cm⁻¹, 2360 ± 2 cm⁻¹, 2342 ± 2 cm⁻¹, 1760 ± 2 cm⁻¹, 1722 ± 2 cm⁻¹, 1696 ± 2 cm⁻¹, 1611 ± 2 cm⁻¹, 1524 ± 2 cm⁻¹, 1479 ± 2 cm^{- 1}, 1446 ± 2 cm⁻¹, 1426 ± 2 cm⁻¹, 1394 ± 2 cm⁻¹, 1365 ± 2 cm⁻¹, 1336 ± 2 cm⁻¹, 1295 ± 2 cm⁻¹, 1268 ± 2 cm⁻¹, 1256 ± 2 cm⁻¹, 1212 ± 2 cm⁻¹, 1191 ± 2 cm⁻¹, 1172 ± 2 cm⁻¹, 1155 ± 2 cm⁻¹, 1136 ± 2 cm⁻¹, 1099 ± 2 cm⁻¹, 1028 ± 2 cm⁻¹, 962 ± 2 cm⁻¹, 872 ± 2 cm⁻¹, 838 ± 2 cm⁻¹, 823 ± 2 cm⁻¹, 775 ± 2 cm⁻¹, 753 ± 2 cm⁻¹, and 654 ± 2 cm⁻¹. Details regarding the IR measurement are provided herein below.
C14. The cocrystal of any preceding item, which is obtainable or obtained by a process comprising a step of cocrystallizing N-[2-[(1S)-1-(3-ethoxy-4-methoxyphenyl)-2-(methylsulfonyl) ethyl]-1,3-dioxo-2,3-dihydro-1H-isoindol-4-yl]acetamide and 1,3,7-trimethylpurine-2,6-dione in the presence of methanol.
C15. The cocrystal of any preceding item, which is characterized by having a dissolution profile as shown in Figure 8.
C16. A mixture of cocrystals of N-[2-[(1S)-1-(3-ethoxy-4-methoxyphenyl)-2-(methylsulfonyl)ethyl]-1,3-dioxo-2,3-dihydro-1H-isoindol-4-yl]acetamide and 1,3,7-trimethylpurine-2,6-dione, comprising at least 50 wt.-%, based on the total weight of the mixture, of the cocrystal of any of items 1-15.

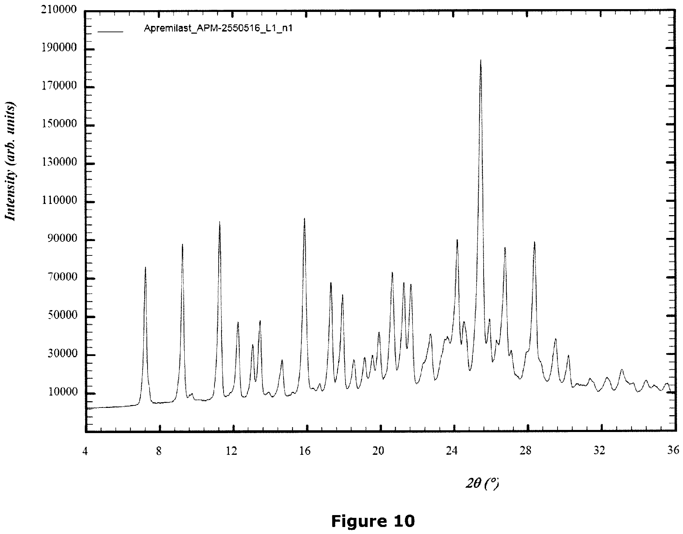

In a further aspect, the invention refers to 4-hydroxybenzoic acid cocrystals:
H1. Cocrystal of N-[2-[(1S)-1-(3-ethoxy-4-methoxyphenyl)-2-(methylsulfonyl)ethyl]-1,3-dioxo-2,3-dihydro-1H-isoindol-4-yl]acetamide and 4-hydroxybenzoic acid.
H2. The cocrystal of item H1, wherein the molar ratio of N-[2-[(1S)-1-(3-ethoxy-4-methoxyphenyl)-2-(methylsulfonyl) ethyl]-1,3-dioxo-2,3-dihydro-1H-isoindol-4-yl]acetamide to 4-hydroxybenzoic acid is about 1:2.
H3. The cocrystal of item H1 or H2, having an X-ray powder diffraction pattern with peaks at 2-theta angles of 7.2±0.2 degrees 2theta, 9.3±0.2 degrees 2theta, 11.3±0.2 degrees 2theta, 12.3±0.2 degrees 2theta, 15.9±0.2 degrees 2theta, 17.3±0.2 degrees 2theta, 18.0±0.2 degrees 2theta, 20.6±0.2 degrees 2theta, 21.3±0.2 degrees 2theta, 21.7±0.2 degrees 2theta, 24.2±0.2 degrees 2theta, 25.5±0.2 degrees 2theta, 26.8±0.2 degrees 2theta, and 28.4±0.2 degrees 2theta when using Cu-Kα radiation. Details regarding the X-ray powder diffraction measurement are provided herein below.
H4. The cocrystal of any of items H1-H3,
(i) having a single endothermic event in a differential scanning calorimeter plot, preferably with a peak value at 153.40±6 °C, further preferred at 153.40±4 °C, even more preferred at 153.40±6 °C; and/or
(ii) wherein the X-ray powder diffraction pattern has further peaks at 2-theta angles of 13.1±0.2 degrees 2theta, 13.5±0.2 degrees 2theta, 19.9±0.2 degrees 2theta, and 27.9±0.2 degrees 2theta, when using Cu-Kα radiation; and/or
(iii) which is substantially polymorphically pure, and preferably contains less than 5 wt.-% of other crystalline, non-crystalline or amorphous forms of N-[2-[(1S)-1-(3-ethoxy-4-methoxyphenyl)-2- (methylsulfonyl)ethyl]-1,3-dioxo-2,3-dihydro-1H-isoindol-4-yl]acetamide, 4-hydroxybenzoic acid, and cocrystals thereof.
The applied temperature range for the differential scanning calorimeter measurement is 25-250°C. Details regarding the differential scanning calorimeter measurement are provided herein below.
H5. The cocrystal of any of items H1-H4, having an X-ray powder diffraction pattern, wherein the peak at 25.5±0.2 degrees 2theta has the highest intensity (set to 100).
H6. The crystalline form of item H4(ii), wherein the X-ray powder diffraction pattern is free of further peaks having an intensity which is higher than the intensity of the peak at 19.9±0.2 degrees 2theta.
H7. The cocrystal of any of items H1-H6, wherein said X-ray powder diffraction pattern is free of further peaks which have an intensity which is in the range of intensities of the peaks defined in items H3 and H4, in particular the X-ray powder diffraction pattern is free of further peaks having an intensity which is higher than the intensity of the peak at 13.1±0.2 degrees 2theta.
H8. The cocrystal of any of items H1-H7, which is substantially pure, i.e. contains less than 1 area-%, or less than 0.5 area-%, preferably less than 0.1 area-%, of compounds other than N-[2-[(1S)-1-(3-ethoxy-4-methoxyphenyl)-2- (methylsulfonyl)ethyl]-1,3-dioxo-2,3-dihydro-1H-isoindol-4-yl]acetamide and 4-hydroxybenzoic acid as determined by quantitative ¹³C NMR measurement or high performance liquid chromatography (HPLC).
H9. The cocrystal of any of items H1-H8, having a mass loss of less than 1.0% of the total mass of the sample in a thermogravimetric analysis (TGA) upon heating from 25°C to 160°C with the gradient of 7°C/min. Details regarding the TGA measurement are provided herein below.
H10. The cocrystal of any of items H1-H9, having an X-ray powder diffraction pattern with peaks and/or intensities as shown in Table 2 (below) or Figure 10.
H11. The cocrystal of any of items H1-H10, having peaks at 2-theta angles ±0.2 degrees 2theta as shown in the following: 7.2, 9.3, 11.3, 12.3, 13.1, 13.5, 14.7, 15.9, 17.3, 18.0, 18.6, 19.1, 19.5, 19.9, 20.6, 21.3, 21.7, 23.5, 24.2, 24.5, 25.5, 25.9, 26.3, 26.8, 27.9, 28.4, 29.5, and 30.2.
H12. The cocrystal of any of items H1-H12, wherein the peaks recited in item H3 and/or item H4 have intensities or preferred intensities as shown in the following Table 2, preferably wherein the X-ray powder diffraction pattern shows all peaks (±0.2 degrees 2theta) and all intensities or preferred intensities as shown in the following Table 2:

**Table 2.**

| **2-theta(deg)** | **Rel. int. I(a.u.)** | **Preferred intensities Rel. int. I(a.u.)** |
|---|---|---|
| 7.2 | 3-12 | 38 |
| 9.3 | 30-54 | 43 |
| 11.3 | 33-58 | 48 |
| 12.3 | 15-32 | 23 |
| 13.1 | 7-20 | 13 |
| 13.5 | 13-28 | 20 |
| 14.7 | 4-15 | 10 |
| 15.9 | 40-70 | 56 |
| 17.3 | 18-44 | 31 |
| 18.0 | 18-40 | 28 |
| 18.6 | 2-10 | 6 |
| 19.1 | 2-10 | 6 |
| 19.5 | 2-10 | 6 |
| 19.9 | 4-18 | 11 |
| 20.6 | 20-40 | 29 |
| 21.3 | 15-30 | 23 |
| 21.7 | 14-29 | 21 |
| 23.5 | 2-10 | 6 |
| 24.2 | 15-40 | 32 |
| 24.5 | 3-11 | 8 |
| 25.5 | 100 | 100 |
| 25.9 | 4-15 | 9 |
| 26.3 | 2-10 | 6 |
| 26.8 | 27-47 | 37 |
| 27.9 | 5-18 | 11 |
| 28.4 | 30-50 | 40 |
| 29.5 | 8-24 | 16 |
| 30.2 | 3-11 | 8 |

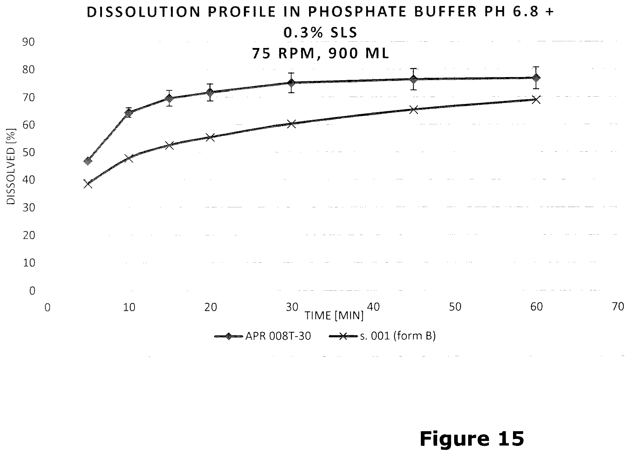

H13. The cocrystal of any of items H1-H12, having an infrared spectrum comprising peaks at wavenumbers of 3368± 2 cm⁻¹, 2999± 2 cm⁻¹, 2362± 2 cm⁻¹, 1760± 2 cm⁻¹, 1693± 2 cm⁻¹, 1668± 2 cm⁻¹, 1609± 2 cm⁻¹, 1519± 2 cm⁻¹, 1479± 2 cm⁻¹, 1426± 2 cm⁻¹, 1393± 2 cm⁻¹, 1365± 2 cm⁻¹, 1335± 2 cm⁻¹, 1300± 2 cm⁻¹, 1269± 2 cm⁻¹, 1234± 2 cm⁻¹, 1188± 2 cm⁻¹, 1169± 2 cm⁻¹, 1136± 2 cm⁻¹, 1098± 2 cm⁻¹, 1047± 2 cm⁻¹, 1027± 2 cm⁻¹, 969± 2 cm⁻¹, 873± 2 cm⁻¹, 837± 2 cm⁻¹, 824± 2 cm⁻¹, 775± 2 cm⁻¹, 752± 2 cm⁻¹, 744± 2 cm⁻¹, and 655± 2 cm⁻¹.
H14. The cocrystal of any of items H1-H13, which is obtainable or obtained by a process comprising a step of cocrystallizing N-[2-[(1S)-1-(3-ethoxy-4-methoxyphenyl)-2-(methylsulfonyl) ethyl]-1,3-dioxo-2,3-dihydro-1H-isoindol-4-yl]acetamide and 4-hydroxybenzoic acid in the presence of acetone.
H15. The cocrystal of any of items H1-H14, which is characterized by having a dissolution profile as shown in Figure 15.
H16. A mixture of cocrystals of N-[2-[(1S)-1-(3-ethoxy-4-methoxyphenyl)-2-(methylsulfonyl)ethyl]-1,3-dioxo-2,3-dihydro-1H-isoindol-4-yl]acetamide and 4-hydroxybenzoic acid, comprising at least 50 wt.-%, based on the total weight of the mixture, of the cocrystal of any of items H1-15.

The invention further relates to pharmaceutical compositions or dosage forms as well as the use of the cocrystals:
1. Pharmaceutical composition or dosage form comprising the cocrystal of any of the preceding items, or the mixture of cocrystals of any of the preceding items.
2. The pharmaceutical dosage form of item 1, which is a solid oral dosage form.
3. The pharmaceutical composition or dosage form of item 1 or 2, which comprises at least one pharmaceutically acceptable excipient.
4. Pharmaceutical composition or dosage form of any of items 1-3 for use in a method of treating a disease or disorder selected from the group consisting of: psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusion body myositis; erosive osteoarthritis; interstitial cystitis; hepatitis; endometriosis; radiculopathy; and pyoderma gangrenosum.
5. Pharmaceutical composition or dosage form of any of items 1-3 for use in a method of treating or preventing a disease or disorder selected from the group consisting of: HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic re perfusion injury; meningitis; psoriasis; psoriatic arthritis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.
6. Use of a cocrystal of any of the preceding items, or a mixture of cocrystals of any of the preceding items, for the preparation of a pharmaceutical composition or dosage form.
7. Use of a cocrystal of any of the preceding items, as a starting material for preparing an oral solid pharmaceutical dosage form comprising said cocrystal.
8. Method of treating a patient by administering a cocrystal of any of the preceding items, or a mixture of cocrystals of any of the preceding items to a patient in need thereof, in particular a patient having a disease as recited in items 4 and/or 5.

The step of formulating the cocrystals into a dosage form may be carried out by applying techniques known in the art, in particular as described herein. For example, the cocrystals can be formulated into tablets by using direct compression, granulation processes, and spraying processes as described above. Examples are also given in the experimental part herein.

Exemplary pharmaceutically acceptable excipients which can be contemplated for use in the present invention are described in the following:

Binding agents are excipients which increase the adhesion of the active agents and excipients during granulation. Examples of binding agents are: sugars, such as sorbitol, glucose, fructose, disaccharides as saccharose, polysaccharides; acacia gum; cellulose derivatives, such as soluble celluloses like microcrystalline cellulose, methylcellulose, hydroxypropylmethylcellulose and hydroxypropylcellulose; tragacanth; polyvinylpyrrolidone, such as polyvinylpyrrolidone-vinyl acetate copolymer, or N-vinylpyrrolidone; sodium alginate and alginate derivatives and gelatin.

Disintegrants are excipients which can take up water and swell and thus improve disintegration of a tablet or granules. Examples of disintegrants are: crospovidone, croscarmellose sodium; starch (paste and pre-gelatinized), such as sodium starch glycolate, methacrylic acid polymer with divinylbenzene, potassium salt, Maltodextrin; croscarmellose sodium and low substituted hydroxypropylcellulose and crospovidone.

Lubricants are excipients which reduce the friction between excipients and compression tooling surfaces. Examples of lubricants are magnesium stearate, magnesium fumarate, fumaric-acid, talc, steraric acid and sodium stearylfumarate.

Fillers are excipients which increase the volume of e.g. tablets. Examples of fillers are mannitol, celluloses such as microcrystalline cellulose, synthetic polymers, Ca-phosphate, inorganic calcium salts, maize starch, polyols and pregelatinized starch.

A polymer, or a plurality of polymers may be used for coating tablets, including for example cellulose derivatives, e.g. hydroxypropylmethylcellulose, polyvinylpyrrolidones, polyethyleneglycols, polyvinylalcohols, acrylates, such as polymethacrylate, cyclodextrins and copolymers and derivatives thereof, including for example polyvinylpyrolidine-vinylacetate. In some preferred embodiments the polymer or the plurality of polymers are pH dependent enteric polymers. Such polymers include cellulose derivatives, e.g. cellulose acetate phthalate, hydroxypropyl methyl cellulose phthalates, hydroxypropyl methyl acetate succinate, hydroxypropyl methyl cellulose acetate, carboxymethylcellulose or a salt thereof, e.g., the sodium salt, cellulose acetate trimellitate, hydroxypropylcellulose acetate phthalate, or polymethylacrylates, e.g., Eudragit®S.

Surfactants include but are not limited to, sorbitan fatty esters, polyoxyethylene sorbit esters, sodium laurylsulfate, sodium docedylbenzenesulfate, dioctyl sodium sulfosuccinate, sodium stearate, EDTA or vitamin E or tocol derivates.

Most preferred for preparing pharmaceutical compositions/dosage forms is the use of one or more, preferably all, of lactose monohydrate, microcrystalline cellulose, magnesium stearate and croscarmellose sodium.

Other excipients are known in the art and can be chosen by a skilled person depending on their function.

### Measuring instruments, conditions and protocols:

### UPLC

Purity and assay analysis can be carried out using Ultra Performance Liquid Chromatograph Waters Acquity I-Class equipped with PDA detector. The analysis can be conducted using Waters Acquity UPLC BEH C18 1.7µm, 2.1x100 mm column, at a temperature of 60°C. The mobile phases were acetonitrile and 0.1 %(v/v) ortho-phosphoric acid in water used in gradient.

### GC

Determination of residual solvents can be carried out using Perkin Elmer Clarus 680 Gas Chromatograph with flame-ionization detector (FID) with carrier gas split, equipped with TurboMatrix 110 Trap a headspace autosampler. The capillary column 60 m x 0.32mm ID x 1.8µm and stationary phase (6%)-cyanopropyl-phenyl-(94%)-dimethylsiloxane DB-624 Agilent Technologies at the temperature gradient can be used.

### XRPD

The analysis of the crystals by X-Ray diffraction was performed using X-ray Diffractometer Rigaku MiniFlex 600 in Bragg-Brentano geometry Cu Kα radiation, D/teX Ultra Detector in a range of 3-36° of 2θ angle. The wavelength of the Cu-Kα radiation used herein is λ=1.5406 Angstrom. The patterns were recorded at a tube voltage of 40 kV, tube current of 15 mA, applying a step size of 0.013° 2° with 80s per step.

### DSC

DSC analysis were carried out using DSC 1 Mettler Toledo Stare thermal analysis system at the temperature range of 25-250°C with the gradient of 5°C/min. Melting, aluminum crucible with the capacity of 40µl were used. Nitrogen was used as carried gas with the flow of 50ml/min.

### TGA

TGA analysis were carried out using TGA/SDTA85 Mettler Toledo Stare thermal analysis system at the temperature range of 25-220°C with the gradient of 7°C/min. Melting, aluminum crucible with the capacity of 100µl were used. Nitrogen was used as carried gas with the flow of 50ml/min.

### DVS

Water Vapour Sorption isotherms were obtained using a Dynamic Vapour Sorption apparatus (model: DVS - Advantage, supplier: Surface Measurement Systems Limited). The DVS apparatus consists of an ultra-microbalance housed inside a temperature-controlled cabinet. The sample was placed into a DVS sample pan under a stream of nitrogen (200 sccm) at 25°C. The humidity was increased ramping in 10% RH (relative humidity) steps from close to 0% RH to 90% RH (sorption phase). Next the humidity was decreased in a similar fashion for the desorption phase. The sample was held at each RH step for mass stabilization (dm/dt 0.02 %/min) but not less than 10 minutes. Two cycles of sorption and desorption were investigated.

### Dissolution studies

Dissolution studies were performed using a Hanson SR8PLUS dissolution apparatus, according to European Pharmacopoeia apparatus, paddle method. The samples were tested in 900mL pH 6.8 with 0.3% SLS at 37.0°C +/- 0.5°C and the paddle rotation speed was set at 75 rpm. Dissolution study was conducted for 60min and samples were analyzed using HPLC method.

### IR measurements

IR analysis were performed on FT-IR Thermo Nicolet Avatar 370 Spectrometer in ATR method and PIKE TECHNOLOGIES Smart MIRacleTM Single Reflection HATR module.

### Stability test

A test for stability against humidity can be performed as follows: The sample is exposed to a relative humidity of 75% (+/- 5%) at 50°C (+/- 2°C) for 2 weeks. The expression "polymorphically stable" means that no conversion to another crystalline form occurs, as determined by XRPD.

The equilibrium relative humidity of a sample is measured by determining the relative humidity in % in the air above a test sample, after establishment of a humidity equilibrium in a closed/open system at a constant temperature.

### HPLC method

Injection Volume: 0.5, 0.2 µl;
Run Time: 15.0 minutes;
Column: Symmetry C18; 250x4,6mm; 5µm, Vial: 1:A,1, 1:c,7, 1:c,8;
Mobile phase: gradient: acetonitrile : buffer (1 vol. TEA and 150 vol. 2,7g/l KH₂PO₄, pH2,0 adjust with H₃PO₄);
Column temp: 25°C; and
Flow: 1 ml/min.

The following examples describe the present invention in detail, but are not to be construed to be in any way limiting for the present invention.

### Example 1 -Preparation of N-[2-[(1S)-1-(3-ethoxy-4-methoxyphenyl)-2-(methylsulfonyl)ethyl]-1,3-dioxo-2,3-dihydro-1H-isoindol-4-yl]acetamide (APM)

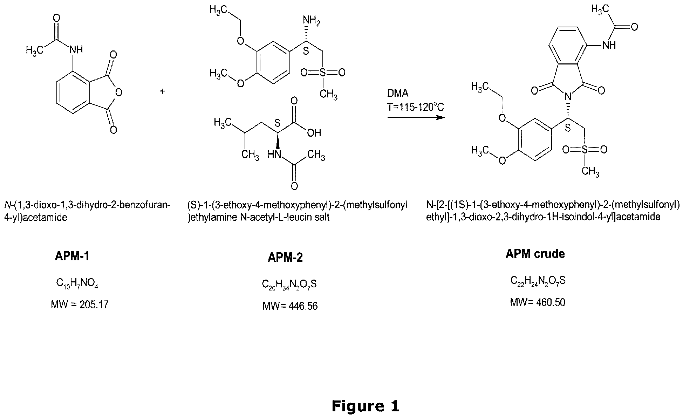

APM-1 (see Figure 1) (9.65g) was dissolved in dimethylacetamide (100ml) at ambient temperature. Then APM-2 (see Figure 1) (20g) was suspended and dimethylacetamide (100 ml) was added. After warming up of the suspension to a temperature of 110-120°C under flow of inert gas, the reaction mixture was stirred between 2-4 hours.

When the reaction was finished (after 2-4h at 110-120°C) the solution was cooled down to a temperature of 20-25°C. To the solution, ethyl acetate (180ml) and water (180ml) were added and the reaction mass was stirred for 10 min at 20-30°C. After separation, the ethyl acetate phase was left and the water phase was extracted with ethyl acetate (2x50ml). The combined ethyl acetate phases was washed with 8% solution NaHCO₃ (2x50ml), 1N HCl (2x50ml), and water (1x50ml). The organic solvent was evaporated under reduced pressure (200-30mbar) at 35°C giving compound APM=Apremilast.

### Examples related to the cocrystallization of Apremilast with caffeine

### Example 2

To the reactor charged with APM 4.75g of caffeine in 125ml of MeOH was added. The mixture was stirred for 16h at room temperature. Obtained solid was filtered under reduced pressure and dried at 50°C and gave product APM-CAF (as confirmed by XRPD and DSC) with a purity of above 99.90% (70-80% yield).

### Example 3

4.75g of caffeine was suspended in 125ml of MeOH, then 10g of APM was added and stirred for 16h at room temperature. Obtained solid was filtered under reduced pressure and dried at 50 °C and gave product APM-CAF (as confirmed by XRPD and DSC) with a purity of above 99.90% (80-90% yield).

### Example 4

4.75g of caffeine and 10g of APM was charged into ball mill and stirred for 3h in a presence of few drops of methanol. After 3h of milling obtained product was dried at 50°C gave product APM-CAF (as confirmed by XRPD and DSC) with a purity of above 99.90% (95-99% yield).

### Example 5 - Characterization of cocrystals of Apremilast with caffeine

Crystalline Apremilast and caffeine cocrystals can be characterized by a X-ray powder diffraction pattern having peaks at 7.5, 11.3, 16.3, 17.7, 19.3, 21.4, 22.4, 24.7, 26.3 and 27.5 degrees two theta, in particular an X-ray powder diffraction pattern as shown in Figure 3.

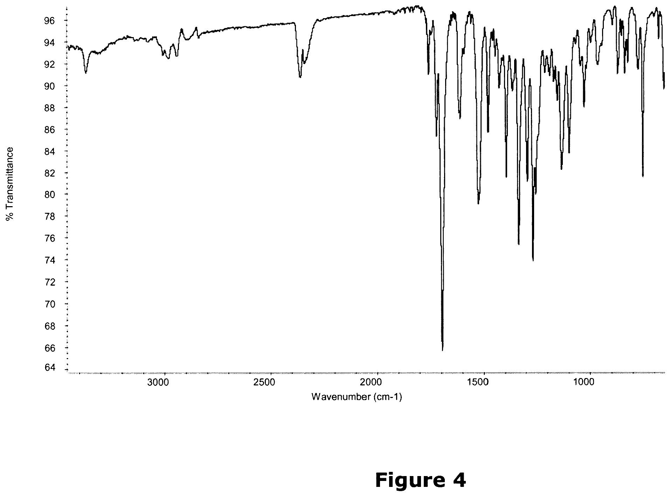

IR can be used to identify the nature of the cocrystal formation. In the IR spectrum of cocrystal shifts in the frequencies of -C=N- stretch and in =C-H out-of-plane bands which appeared at 1668 and 969 and 873 cm⁻¹ respectively. These shifts suggested that both the N atom of -C=N- and H atom of = C-H group of caffeine were involved in some kind of hydrogen bond interactions. The IR spectrum of Apremilast cocrystal with caffeine is shown in Figure 4.

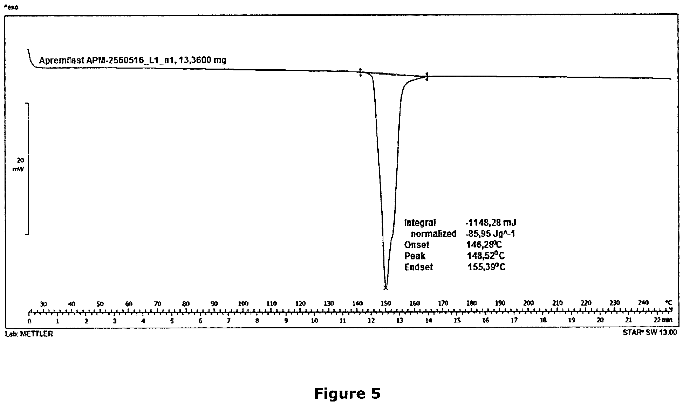

Crystalline Apremilast and caffeine cocrystals may be also characterized by thermal analysis. A representative DSC plot for Apremilast and caffeine cocrystals is shown in Figure 5. The DSC plot comprises an endothermic event with an onset temperature of [148.52±6 °C].

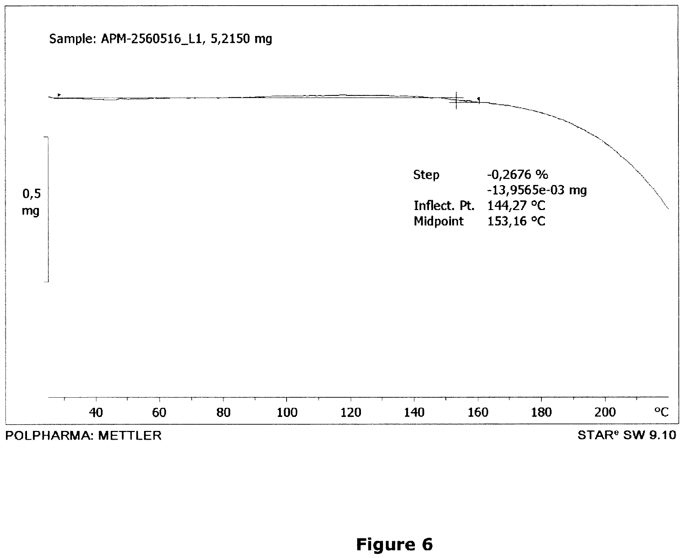

A representative TGA plot for Apremilast and caffeine cocrystals is shown in Figure 6. The TGA plot comprises a mass loss of less than about 0.5%, e.g., less than 0.3%, of the total mass of the sample upon heating from about 25°C to about 160°C.

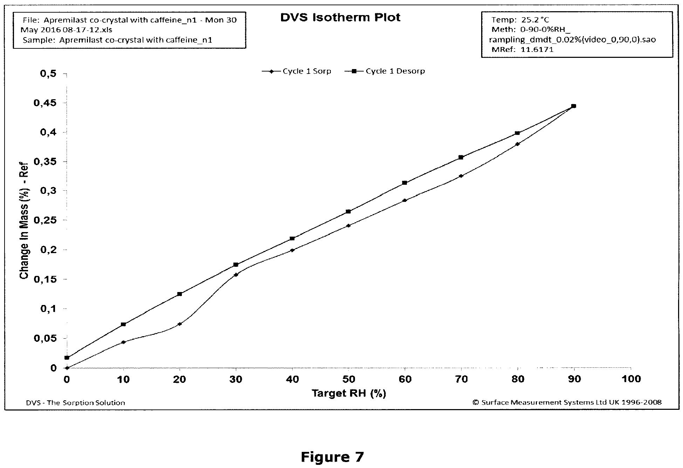

Apremilast and caffeine cocrystals may be characterized by moisture sorption analysis. A representative moisture sorption isotherm plot is shown in Figure 7. When the RH is increased from about 0% to about 95% RH, a mass change of less than about 0.5%, e.g., about 0.45%, of the starting at about 0% RH occurs. Apremilast and caffeine cocrystal is slightly hygroscopic.

Figure 3 shows a representative XRPD pattern of Apremilast and caffeine cocrystal, wherein the data are summarized in Table 3:

**Table 3.**

| **No.** | **2-theta(deg)** | **Rel. int. I(a.u.)** |
|---|---|---|
| 1 | 7.469(3) | 64.87 |
| 2 | 9.074(6) | 10.22 |
| 3 | 9.664(6) | 7.87 |
| 4 | 11.253(3) | 45.03 |
| 5 | 11.932(5) | 15.16 |
| 6 | 13.162(7) | 5.51 |
| 7 | 13.9752(15) | 22.44 |
| 8 | 15.259(9) | 26.60 |
| 9 | 16.340(4) | 49.07 |
| 10 | 17.701(4) | 75.35 |
| 11 | 19.291(13) | 33.56 |
| 12 | 20.186(15) | 15.77 |
| 13 | 20.70(2) | 11.69 |
| 14 | 21.353(10) | 32.94 |
| 15 | 22.39(2) | 34.22 |
| 16 | 23.39(3) | 21.45 |
| 17 | 24.697(14) | 34.53 |
| 18 | 25.418(18) | 20.11 |
| 19 | 26.290(8) | 100.00 |
| 20 | 27.456(14) | 36.86 |
| 21 | 28.945(12) | 31.25 |
| 22 | 29.724(14) | 9.38 |
| 23 | 30.44(5) | 5.94 |
| 24 | 33.63(3) | 5.75 |

Figure 4 shows a representative IR spectrum of Apremilast and caffeine cocrystal, wherein the data are summarized in the following: Wavenumber [cm⁻¹]: 3369, 2981, 2939, 2360, 2342, 1760, 1722, 1696, 1611, 1524, 1479, 1446, 1426, 1394, 1365, 1336, 1295, 1268, 1256, 1212, 1191, 1172, 1155, 1136, 1099, 1028, 962, 872, 838, 823, 775, 753, and 654.

### Example 6 - Dissolution properties of cocrystals of Apremilast with caffeine

Table 4 shows the dissolution profile of Apremilast cocrystal with caffeine and Form B in buffer pH 6.8 + 0.3% SLS (see also Figure 8).

**Table 4.**

| | **APREMILAST** | | | |
|---|---|---|---|---|
| **Tablets** | s. 001 (Form B) | | s. 009 (caffeine cocrystal) | |
| | Assay: | 95.1% | Assay: | 93.7% |
| **n=3** | Mean | RSD | Mean | RSD |

| **Time [min]** | | | | |
|---|---|---|---|---|
| 5 | 38.6 | 4.3 | 42.7 | 1.1 |
| 10 | 47.8 | 4.5 | 65.5 | 2.2 |
| 15 | 52.5 | 4.0 | 75.4 | 1.6 |
| 20 | 55.4 | 5.0 | 81.4 | 2.0 |
| 30 | 60.3 | 4.8 | 89.8 | 2.3 |
| 45 | 65.4 | 6.3 | 94.2 | 2.2 |
| 60 | 68.9 | 6.4 | 96.4 | 2.0 |

### Example 7 - Stability tests with tablets containing Form B (prior art)

Tablets comprising Form B were prepared as follows:

Apremilast, lactose monohydrate, microcrystalline cellulose, croscarmellose sodium were passed through a 0.425 mm sieve. The ingredients from the sieving step are mixed in a blender for 15 min at 25 RPM (rotations per minute). Magnesium stearate is added and the mixture is mixed for 5 min at 12 RPM. The resulting blend is compressed using a single punch machine equipped with an oval punch (14x8mm) at a compression force of 7 kN. The composition of the tablets is shown in Table 5.

**Table 5. (Sample 001)**

| **Ingredient** | **mg/tabl** | **%/tabl** |
|---|---|---|
| Apremilast (Form B) | 30.00 | 10.00 |
| Lactose monohydrate | 171.00 | 57.00 |
| Microcrystalline cellulose | 85.50 | 28.50 |
| Croscarmellose sodium | 11.25 | 3.75 |
| Magnesium stearate | 2.25 | 0.75 |
| total: | 300.00 | 100.00 |

Stability results after 2 weeks (conditions either closed or open):

**Table 6.**

| **Sample 001** | |
|---|---|
| **initial** | **Form B** |
| **50°C/75%RH close** | not changed |
| **50°C/75%RH open** | not changed |
| **RT/70-75%RH open** | not changed |

### Example 8 - Stability tests with tablets containing Apremilast cocrystal with caffeine

Sample 009 comprising Apremilast cocrystal with caffeine was prepared as follows:

Apremilast cocrystal with caffeine, lactose monohydrate, microcrystalline cellulose, croscarmellose sodium were passed through a 0.425 mm sieve. The ingredients from the previous step were mixed in a blender for 15 min at 25 RPM. Magnesium stearate was added and the resulting mixture was mixed for 5 min at 12 RPM. The resulting blend was compressed using a single punch machine equipped with oval punch (14x8mm) at a compression force of 7 kN

**Table 7.**

| **Ingredient** | **mg/tabl** | **%/tabl** |
|---|---|---|
| Apremilast (Apremilast cocrystal with caffeine) | 34,50 | 11,50 |
| Lactose monohydrate | 168,00 | 56,00 |
| Microcrystalline cellulose | 84,00 | 28,00 |
| Croscarmellose sodium | 11,25 | 3,75 |
| Magnesium stearate | 2,25 | 0,75 |
| total: | 300,00 | 100,00 |

Stability results after 2 weeks:

**Table 8.**

| **Sample 009** | |
|---|---|
| **initial** | **Apremilast cocrystal with caffeine** |
| **50°C/75%RH close** | not changed |
| **50°C/75%RH open** | not changed |
| **RT/70-75%RH open** | not changed |

### Examples related to the cocrystallization of Apremilast with 4-hydroxybenzoic acid

### Example 9

To the reactor charged with APM 4.5g of 4-hydroxybenzoic acid in 125ml of acetone-water was added. The mixture was stirred for 16h at room temperature. Obtained solid was filtered under reduced pressure and dried at 50°C gave product APM-4HB (as confirmed by XRPD and DSC) with a purity of above 99.90% (70-80% yield).

### Example 10

4.5g of 4-hydroxybenzoic acid was suspended in 125ml of acetone-water, then 10g of APM was added and stirred for 16h at room temperature. Obtained solid was filtered under reduced pressure and dried at 50°C gave product APM-4HB (as confirmed by XRPD and DSC) with a purity of above 99.90% (80-90% yield).

### Example 11

2.25g of 4-hydroxybenzoic acid and 10g of APM was charged into ball mill and stirred for 3h in a presence of few drops of acetone. After 3h of milling, the obtained product was dried at 50°C and gave product APM-4HB (as confirmed by XRPD and DSC) with a purity of above 99.90% (95-99% yield).

### Example 12 - Characterization of cocrystals of Apremilast with 4-hydroxybenzoic acid

Crystalline Apremilast and 4-hydroxybenzoic acid cocrystal can be characterized by X-ray powder diffraction pattern having peaks at 7.2, 9.3, 11.3, 12.3, 17.3, 18.0, 20.6, 21.3, 21.7, 24.2, 25.5, 26.8, and 28.4 degrees two theta, an X-ray powder diffraction pattern as depicted in Figure 10. The XRPD data are summarized in Table 9:

**Table 9.**

| **No.** | **2-theta(deg)** | **Rel. int. I(a.u.)** |
|---|---|---|
| 1 | 7.243(3) | 38.43 |
| 2 | 9.267(4) | 42.94 |
| 4 | 11.294(4) | 47.93 |
| 5 | 12.252(11) | 22.50 |
| 6 | 13.053(6) | 13.18 |
| 7 | 13.454(5) | 19.56 |
| 8 | 14.659(8) | 9.67 |
| 9 | 15.893(4) | 55.81 |
| 10 | 17.318(7) | 30.97 |
| 11 | 17.967(7) | 28.40 |
| 12 | 18.562(16) | 5.97 |
| 13 | 19.11(4) | 5.86 |
| 14 | 19.54(5) | 6.48 |
| 15 | 19.92(3) | 11.04 |
| 16 | 20.621(7) | 29.29 |
| 17 | 21.26(3) | 23.30 |
| 18 | 21.66(3) | 20.97 |
| 19 | 23.50(7) | 6.38 |
| 20 | 24.17(4) | 32.14 |
| 21 | 24.54(11) | 7.76 |
| 22 | 25.481(7) | 100.00 |
| 23 | 25.916(8) | 9.42 |
| 24 | 26.29(3) | 5.88 |
| 25 | 26.773(16) | 37.04 |
| 26 | 27.93(3) | 11.20 |
| 27 | 28.353(10) | 40.08 |
| 28 | 29.496(13) | 15.95 |
| 29 | 30.194(5) | 8.34 |

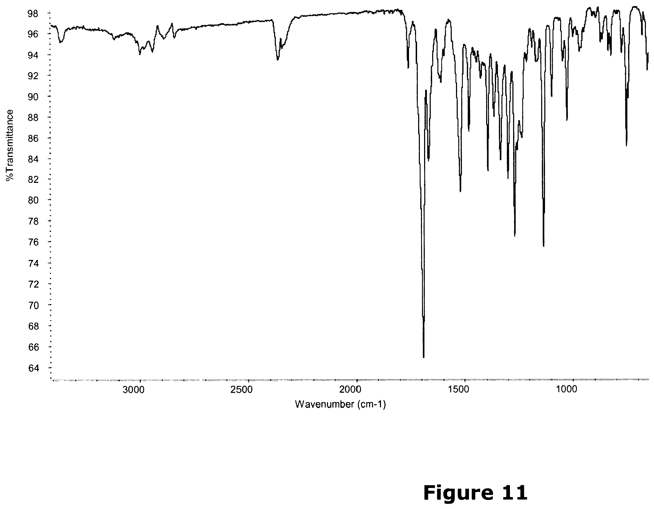

IR can be used to identify the nature of the cocrystal formation. In the IR spectrum of cocrystal systems involving carboxylic acid groups, the energy of the antisymmetric stretching mode of the carbonyl group could be used to identify the nature of the species formed. When the free acid absorption band (at a range of 1680-1690 cm⁻¹) disappeared entirely and became replaced by the corresponding anion band (at a range of 1550-1600 cm⁻¹), a salt has been formed. When the free acid absorption band undergoes a small shift towards higher energy (at range of 1700-1730 cm⁻¹), one has observed the formation of a cocrystal. In IR spectrum of Apremilast cocrystal showed in Figure 11 carboxylic acid group absorption band is shifted from the 1671 to 1696 cm⁻¹ confirming hydrogen bond interactions. The IR data are summarized as follows: Wavenumber [cm⁻¹]: 3368, 2999, 2362, 1760, 1693, 1668, 1609, 1519, 1479, 1426, 1393, 1365, 1335, 1300, 1269, 1234, 1188, 1169, 1136, 1098, 1047, 1027, 969, 873, 837, 824, 775, 752, 744, and 655.

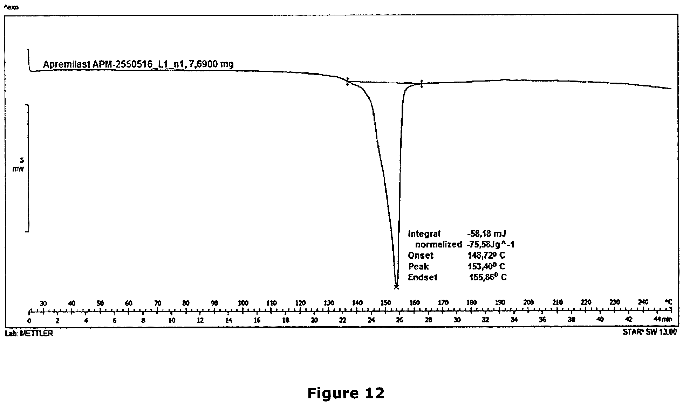

Crystalline Apremilast and 4-hydroxybenzoic acid cocrystal may be also characterized by thermal analysis. A representative DSC plot for Apremilast and 4-hydroxybenzoic acid cocrystal is shown in Figure 12. DSC plot of Apremilast and 4-hydroxybenzoic acid cocrystal comprising an endothermic event with an onset temperature of about 148°C±2°C.

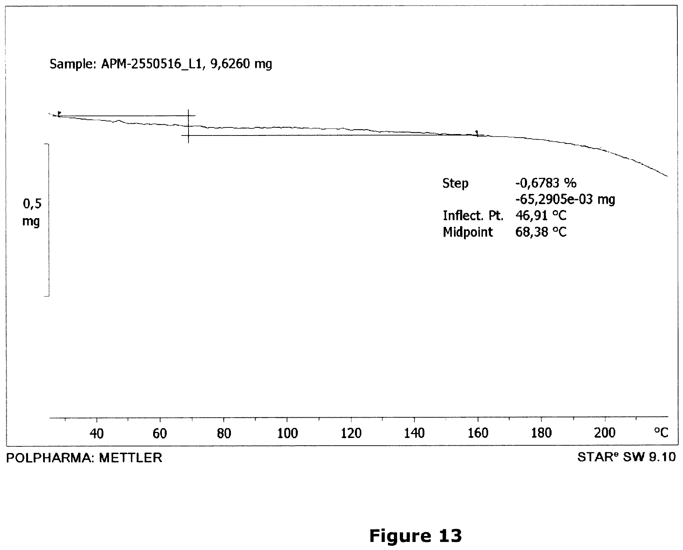

A representative TGA plot for Apremilast and 4-hydroxybenzoic acid cocrystal is shown in Figure 13. TGA plot comprising a mass loss of less than about 1.0%, e.g., less than 0.7%, of the total mass of the sample upon heating from about 25°C to about 160°C.

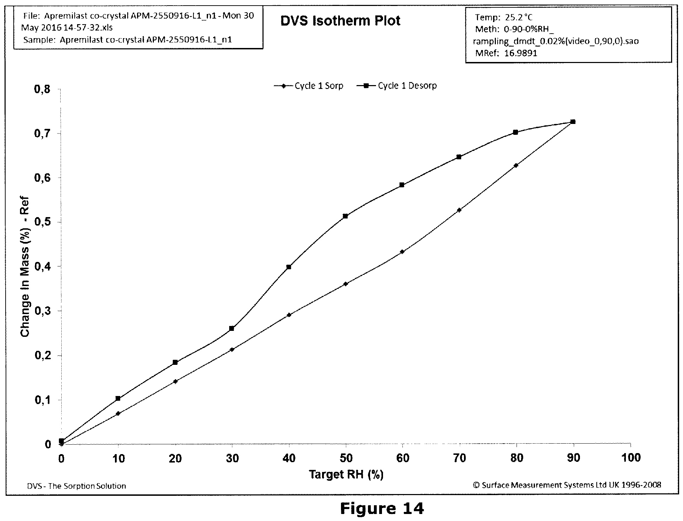

Apremilast and 4-hydroxybenzoic acid cocrystal may be characterized by moisture sorption analysis. A representative moisture sorption isotherm plot is shown in Figure 14, when the RH is increased from about 0% to about 95% RH exhibiting a mass change of less than about 1%, e.g., about 0.7%, of the starting about 0% RH. Apremilast and 4-hydroxybenzoic acid cocrystal is slightly hygroscopic.

### Example 13 - Dissolution properties of cocrystals of Apremilast with 4-hydroxybenzoic acid

Figure 15 shows a dissolution profile of cocrystals of Apremilast with 4-hydroxybenzoic acid of the present invention compared with that of prior art Form B (see Example 7), wherein the new cocrystal of Apremilast with 4-hydroxybenzoic acid exhibited a higher dissolution rate.

The dissolution profile of Apremilast cocrystal with 4-hydroxybenzoic acid and Form B in buffer pH 6.8 + 0.3% SLS is also shown in Table 10:

**Table 10.**

| | **APREMILAST** | | | |
|---|---|---|---|---|
| **Tablets** | s. 001 (Form B) | | s. 008 (4-hydroxybenzoic acid cocrystal) | |
| | Assay: | 95.1% | Assay: | 93.7% |
| **n=3** | Mean | RSD | Mean | RSD |

| **Time [min]** | | | | |
|---|---|---|---|---|
| 5 | 38.6 | 4.3 | 46.8 | 1.1 |
| 10 | 47.8 | 4.5 | 64.5 | 2.7 |
| 15 | 52.5 | 4.0 | 69.6 | 4.1 |
| 20 | 55.4 | 5.0 | 71.7 | 4.3 |
| 30 | 60.3 | 4.8 | 75.1 | 4.6 |
| 45 | 65.4 | 6.3 | 76.3 | 5.1 |
| 60 | 68.9 | 6.4 | 76.7 | 5.2 |

### Example 14 - Stability tests with tablets containing Apremilast cocrystal with 4-hydroxybenzoic acid

Sample 008 comprising Apremilast cocrystal with 4-hydroxybenzoic acid was prepared as follows: Apremilast cocrystal with 4-hydroxybenzoic acid, lactose monohydrate, microcrystalline cellulose, croscarmellose sodium were passed through a 0.425 mm sieve. The ingredients from the previous step were mixed in a blender for 15 min at 25 RPM. Magnesium stearate was added and the resulting mixture was mixed for 5 min at 12 RPM. The resulting blend was compressed using a single punch machine equipped with oval punch (14x8mm) at a compression force of 7 kN, see Table 11.

**Table 11.**

| **Ingredient** | **mg/tabl** | **%/tabl** |
|---|---|---|
| Apremilast (Apremilast cocrystal with 4-hydroxybenzoic acid) | 30,00 | 10,00 |
| Lactose monohydrate | 171,00 | 57,00 |
| Microcrystalline cellulose | 85,50 | 28,50 |
| Croscarmellose sodium | 11,25 | 3,75 |
| Magnesium stearate | 2,25 | 0,75 |
| total: | 300,00 | 100,00 |

Stability results after 2 weeks:

**Table 12.**

| **Sample 008** | |
|---|---|
| **initial** | **Apremilast cocrystal with 4-hydroxybenzoic acid** |
| **50*C/75%RH close** | not changed |
| **50°C/75%RH open** | not changed |
| **RT/70-75%RH open** | not changed |

### Example 15 - Summary on cocrystallization experiments

| **No** | **Main changes** | **Result** |
|---|---|---|
| 1 | 0.18g of saccharin was suspended in 5ml of MeOH, then 0.4g of APM was added and stirred for 16h. Solid was filtered and dried at 50°C | Form B |
| | | No cocrystal formation |
| 2 | 0.19g of caffeine was suspended in 5ml of MeOH, then 0.4g of APM was added and stirred for 16h. Solid was filtered and dried at 50°C | Different XRPD |
| | | Cocrystal formation |
| 3 | 0.13g of citric acid was suspended in 5ml of MeOH, then 0.4g of APM was added and stirred for 16h. Solid was filtered and dried at 50°C | Form B |
| | | No cocrystal formation |
| 4 | 0.14g of acetylsalicylic acid was suspended in 5ml of MeOH, then 0.4g of APM was added and stirred for 16h. Solid was filtered and dried at 50°C | Form B |
| | | No cocrystal formation |
| 5 | 0.18g of fructose acid was suspended in 5ml of MeOH, then 0.4g of APM was added and stirred for 16h. Solid was filtered and dried at 50°C | Form B |
| | | No cocrystal formation |
| 6 | 0.12g of fumaric acid was suspended in 5ml of MeOH, then 0.4g of APM was added and stirred for 16h. Solid was filtered and dried at 50°C | Form B |
| | | No cocrystal formation |
| 7 | 0.18g of ascorbic acid was suspended in 5ml of MeOH, then 0.4g of APM was added and stirred for 16h. Solid was filtered and dried at 50°C | Form B |
| | | No cocrystal formation |
| 8 | 0.18g of mannitol was suspended in 5ml of MeOH, then 0.4g of APM was added and stirred for 16h. Solid was filtered and dried at 50°C | Form B |
| | | No cocrystal formation |
| 9 | 0.4g of APM was dissolved in 5ml of acetone and then 0.18g of saccharin was added and stirred for 16h. Solid was filtered and dried at 50°C | Form B |
| | | No cocrystal formation |
| 10 | 0.4g of APM was dissolved in 5ml of acetone and then 0.19g of caffeine was added and stirred for 16h. Solid was filtered and dried at 50°C | Mixture of cocrystal |
| | | and form B |
| 11 | 0.4g of APM was dissolved in 5ml of acetone and then 0.13g of citric acid was added and stirred for 16h. Solid was filtered and dried at 50°C | Form B |
| | | No cocrystal formation |
| 12 | 0.4g of APM was dissolved in 5ml of acetone and then 0.14g of acetylsalicylic acid was added and stirred for 16h. Solid was filtered and dried at 50°C | Form B |
| | | No cocrystal formation |
| 13 | 0.4g of APM was dissolved in 5ml of acetone and 0.18g of 4-hydroxybenzoic acid was added and stirred for 16h. Solid was filtered and dried at 50°C | Different XRPD |
| | | Cocrystal formation |
| 14 | 0.4g of APM was dissolved in 5ml of acetone and 0.18g of fructose acid was added and stirred for 16h. Solid was filtered and dried at 50°C | Form B |
| | | No cocrystal formation |
| 15 | 0.4g of APM was dissolved in 5ml of acetone and 0.12g of fumaric acid was added and stirred for 16h. Solid was filtered and dried at 50°C | Form B |
| | | No cocrystal formation |
| 16 | 0.4g of APM was dissolved in 5ml of acetone and 0.18g of ascorbic acid was added and stirred for 16h. Solid was filtered and dried at 50°C | Form B |
| | | No cocrystal formation |
| 17 | 0.4g of APM was dissolved in 5ml of acetone and 0.18g of mannitol was added and stirred for 16h. Solid was filtered and dried at 50°C | Form B |
| | | No cocrystal formation |
| 18 | Cocrystal with 4-hydroxybenzoic acid *10g scale* | Cocrystal formation confirmed by XRPD and DSC |
| | Stoichiometry: 2:1 (APM : 4-HBA) | Stoichiometry and purity confirmed by HPLC |
| | Tablets were prepared and dissolution profiles checked and compared to form B | Solubility: 42.5µg/ml (RT) |
| 19 | Cocrystal with caffeine | Cocrystal formation confirmed by XRPD and DSC |
| | *10g scale* | Stoichiometry and purity confirmed by HPLC |
| | Stoichiometry: 2:1 (APM : CAFF) | |
| | Tablets were prepared and dissolution profiles checked and compared to form B | Solubility: 20.6µg/ml (RT) |

### Example 16 - 13C CP/MAS NMR experiments

13C CP/MAS NMR experiments with (i) Apremilast, 4-hydroxybenzoic acid and 4-hydroxybenzoic acid cocrystal (Figure 16) and (ii) Apremilast, caffeine and caffeine cocrystal (Figure 17) were performed using the conditions set forth below.

The solid-state cross-polarization magic angle spinning (CPMAS) NMR experiments were performed on a Bruker Avance III 400 spectrometer, at a frequency of 100.61 MHz for 13C, which was equipped with a MAS probehead using 4-mm ZrO2 rotors. A sample of glycine (Gly) was used to set the Hartmann-Hahn condition for 13C nuclei. Spectra use adamantane as a secondary chemical-shift reference at 38.48 and 29.46 ppm from external tetramethylsilane (TMS). Samples were spun ad 8kHz. For pure apremilast sample repetition rate was equal to 10s, for obtained adequate signal-to-noisy ratio we collected 512 scans. Due to long T1 (1 H) relaxation time of caffeine and 4-hydroxybenzoic acid repetition rate was 120s, in those cases 24 scans were enough to obtained adequate signal-to-noisy ratio. For both cocrystals, 2 experiments were performed with a repetition rate equal to 10s and 120s and 512 scans. The spectra were exactly the same for both repetition rates.

### Example 17 - HPLC experiments

The HPLC experiments were performed by applying the conditions described in the Section "Measuring instruments, conditions and protocols" above.

**Cocrystal of Apremilast and 4-hydroxybenzoic acid.**

| Compound | Retention time | Area% |
|---|---|---|
| 4-hydroxybenzoic acid | 2.85 | 3.73 |
| Apremilast | 7.22 | 96.27 |

**Cocrystal of Apremilast and 1,3,7-trimethylpurine-2,6-dione:**

| Compound | Retention time | Area% |
|---|---|---|
| Caffeine | 3.16 | 11.20 |
| Apremilast, | 7.22 | 88.80 |

### Cited literature

EP2276483 B1; US 6,962,940

## Claims

1. Cocrystal of N-[2-((1S)-1-(3-ethoxy-4-methoxyphenyl)-2-(methylsulfonyl) ethyl]-1,3-dioxo-2,3-dihydro-1H-isoindol-4-yl]acetamide and 1,3,7-trimethylpurine-2,6-dione or 4-hydroxybenzoic acid.

2. The cocrystal of claim 1, which is a cocrystal of N-[2-[(1S)-1-(3-ethoxy-4-methoxyphenyl)-2-(methylsulfonyl) ethyl]-1,3-dioxo-2,3-dihydro-1H-isoindol-4-yl]acetamide and 1,3,7-trimethylpurine-2,6-dione, wherein
(i) the molar ratio of N-[2-[(1S)-1-(3-ethoxy-4-methoxyphenyl)-2-(methylsulfonyl) ethyl]-1,3-dioxo-2,3-dihydro-1H-isoindol-4-yl]acetamide to 1,3,7-trimethylpurine-2,6-dione is about 1:2, and/or
(ii) the cocrystal has an X-ray powder diffraction pattern with peaks at 2-theta angles of 7.5±0.2 degrees 2theta, 11.3±0.2 degrees 2theta, 16.3±0.2 degrees 2theta, 17.7±0.2 degrees 2theta, 19.3±0.2 degrees 2theta, 21.4±0.2 degrees 2theta, 22.4±0.2 degrees 2theta, 24.7±0.2 degrees 2theta, 26.3 ±0.2 degrees 2theta, and 27.5 ±0.2 degrees 2theta when using Cu-Kα radiation; and/or
(iii) the cocrystal has a single endothermic event in a differential scanning calorimeter plot, preferably with a peak value at 148.52±6 °C, further preferred at 148.52±4 °C, even more preferred at 148.52±2 °C.

3. The cocrystal of claim 1 or 2 which is substantially polymorphically pure, and preferably contains less than 5 wt.-% of other crystalline, non-crystalline or amorphous forms of N-[2-[(1S)-1-(3-ethoxy-4-methoxyphenyl)-2- (methylsulfonyl)ethyl]-1,3-dioxo-2,3-dihydro-1H-isoindol-4-yl]acetamide, 1,3,7-trimethylpurine-2,6-dione, and cocrystals thereof.

4. The cocrystal of any preceding claim, having a mass loss of less than 0.5% of the total mass of the sample in a thermogravimetric analysis upon heating from 25°C to 160°C with the gradient of 7°C/min.

5. The cocrystal of claim 1, which is a cocrystal of N-[2-[(1S)-1-(3-ethoxy-4-methoxyphenyl)-2-(methylsulfonyl) ethyl]-1,3-dioxo-2,3-dihydro-1H-isoindol-4-yl]acetamide and 4-hydroxybenzoic acid, wherein
(i) the molar ratio of N-[2-[(1S)-1-(3-ethoxy-4-methoxyphenyl)-2-(methylsulfonyl) ethyl]-1,3-dioxo-2,3-dihydro-1H-isoindol-4-yl]acetamide to 4-hydroxybenzoic acid is about 1:2; and/or
(ii) the cocrystal has an X-ray powder diffraction pattern with peaks at 2-theta angles of 7.2±0.2 degrees 2theta, 9.3±0.2 degrees 2theta, 11.3±0.2 degrees 2theta, 12.3±0.2 degrees 2theta, 17.3±0.2 degrees 2theta, 18.0±0.2 degrees 2theta, 20.6±0.2 degrees 2theta, 21.3±0.2 degrees 2theta, 21.7±0.2 degrees 2theta, 24.2±0.2 degrees 2theta, 25.5±0.2 degrees 2theta, 26.8±0.2 degrees 2theta, and 28.4±0.2 degrees 2theta when using Cu-Kα radiation; and/or
(iii) the cocrystal has a single endothermic event in a differential scanning calorimeter plot, preferably with a peak value at 153.40±6 °C; and/or
(iv) which is substantially polymorphically pure, and preferably contains less than 5 wt.-% of other crystalline, non-crystalline or amorphous forms of N-[2-[(1S)-1-(3-ethoxy-4-methoxyphenyl)-2- (methylsulfonyl)ethyl]-1,3-dioxo-2,3-dihydro-1H-isoindol-4-yl]acetamide, 4-hydroxybenzoic acid, and cocrystals thereof.

6. The cocrystal of claim 5, having a mass loss of less than 1.0% of the total mass of the sample in a thermogravimetric analysis upon heating from 25°C to 160°C with the gradient of 7°C/min.

7. A mixture of cocrystals of N-[2-[(1S)-1-(3-ethoxy-4-methoxyphenyl)-2-(methylsulfonyl)ethyl]-1,3-dioxo-2,3-dihydro-1H-isoindol-4-yl]acetamide and 1,3,7-trimethylpurine-2,6-dione or 4-hydroxybenzoic acid, comprising at least 50 wt.-%, based on the total weight of the mixture, of the cocrystal of any of claims 1-6.

8. Pharmaceutical composition or dosage form comprising the cocrystal of any of claims 1-6, or the mixture of cocrystals of claim 7.

9. The pharmaceutical dosage form of claim 8, which is a solid oral dosage form.

10. The pharmaceutical composition or dosage form of claim 8 or 9, which comprises at least one pharmaceutically acceptable excipient.

11. Pharmaceutical composition or dosage form of any of claims 8-10 for use in a method of treating a disease or disorder selected from the group consisting of: psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusion body myositis; erosive osteoarthritis; interstitial cystitis; hepatitis; endometriosis; radiculopathy; and pyoderma gangrenosum.

12. Pharmaceutical composition or dosage form of any of claims 8-10 for use in a method of treating or preventing a disease or disorder selected from the group consisting of: HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic re perfusion injury; meningitis; psoriasis; psoriatic arthritis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.

13. Use of a cocrystal of any of claims 1-6, or a mixture of cocrystals of claim 7, for the preparation of a pharmaceutical composition or dosage form.
